# EUROPEAN PATENT APPLICATION

(11) **EP 4 656 190 A1**
(43) Date of publication of application: **03.12.2025**
(21) Application number: 24747462.0
(22) Date of filing: 25.01.2024
(51) Int. Cl.: A61K 31/19, A61P 21/00, A23L 33/12, G01N 30/72, G01N 30/88

(54) **COMPOSITION FOR PREVENTING, TREATING OR AMELIORATING SARCOPENIA AND METHOD FOR DIAGNOSING SARCOPENIA**

(30) Priority: 27.01.2023 KR 20230011066
(71) Applicant: Korea Research Institute of Bioscience and Biotechnology, Daejeon 34141 (KR); Aventi Inc., Daejeon 34141 (KR)
(72) Inventor: KWON, Ki-Sun, Daejeon 34141 (KR); KWAK, Ju Yeon, Daejeon 34141 (KR); LEE, Younglang, Daejeon 34141 (KR)
(74) Representative: Dehns
(86) International application number: PCT/KR2024/001206
(87) International publication number: WO 2024/158228

(57) **Abstract**

The present invention relates to a pharmaceutical composition, health functional food composition or health functional food for the prevention, treatment or amelioration of sarcopenia, comprising at least one of one or more short-chain or medium-chain fatty acids, ester(s) thereof, or triglyceride form(s) thereof. Also, the present invention relates to a composition for the diagnosis of a possible sarcopenia group or a sarcopenia group, the composition comprising, as an active ingredient, an agent for measuring the content of one or more short-chain or medium-chain fatty acids in the blood, and relates to a method for providing information for determining a possible sarcopenia group or a sarcopenia group, the method comprising a step of measuring the content of one or more short-chain or medium-chain fatty acids from the blood of a subject.

## Description

### Technical Field

The present invention relates to a pharmaceutical composition, a health functional food composition for sarcopenia, a health functional food, and a diagnostic composition comprising one or more short-chain or medium-chain fatty acids, and relates to a method for diagnosing a possible sarcopenia group or sarcopenia group comprising measuring the content of one or more short-chain or medium-chain fatty acids from the blood of a subject.

### Background Art

A decrease in muscle mass and strength is one of the major characteristics of human aging, and it is a factor that significantly affects all of physical activity ability, lifestyle, and appearance of an individual. A decrease in muscle mass and strength due to aging is one of the symptoms of sarcopenia, which is a disease accompanied by symptoms of a decrease in muscle mass and muscle function as the number and size of muscle fibers constituting the muscle decreases.

Patients with sarcopenia have a decreased ability to perform daily physical activities, such as walking speed or the ability to sit and stand up, due to a lack of muscle mass, making them more prone to frequent falls and thus being exposed to their risk of fall-related accidents. Additionally, since muscles also serve as an energy source in the body by storing glucose in the form of glycogen, it is known that a decrease in muscle mass can lead to fatigue more easily and may worsen chronic diseases.

Meanwhile, the UN classifies a country as an aging society when the proportion of people aged 65 and older exceeds 14% of the total population, and as a super-aged society when it exceeds 20%. According to UN population projections, the proportion of elderly people in developed countries is expected to increase from 13.0% in 1990 to 23.3% in 2030, and in developing countries, it is also projected to more than double, from 4.6% in 1990 to 9.7% in 2030. As the elderly population increases, the number of patients with sarcopenia or those at risk of developing sarcopenia is rising rapidly.

Accordingly, techniques for diagnosing sarcopenia have been studied, and sarcopenia is currently diagnosed by directly measuring muscle mass using imaging techniques such as dual energy x-ray absorptiometry (DEXA) or computed tomography (CT), along with assessments of muscle strength (*e.g*., grip strength) and performance of physical activities (*e.g.*, walking speed, chair stand test, and the short physical performance battery (SPPB)), followed by the interpretation of the scores obtained from these assessments in accordance with the European (EWGSOP) or Asian (AWGS) guidelines.

However, the above-mentioned tests are not only cumbersome and uneconomical as they require expensive medical equipment, but also pose a risk of radiation exposure during these tests. Therefore, there is a growing need to develop accurate, safe, and cost-effective diagnostic tools for sarcopenia as alternatives.

Although exercise and nutrition are known to help delay sarcopenia, there are currently no approved treatments by regulatory agencies such as the FDA or their global counterparts.

### Disclosure of Invention

### Technical Problem

The present invention aims to solve technical challenges for simply and safely diagnosing, preventing, treating, or improving sarcopenia by providing a method for providing information for determining a possible sarcopenia group and a sarcopenia group by measuring the content of one or more short-chain or medium-chain fatty acids in the blood; and a composition comprising said fatty acids for preventing, treating, or improving sarcopenia.

### Solution to Problem

In order to solve the above problems, the present invention provides a pharmaceutical composition, a health functional food composition, and a health functional food for preventing, treating, or improving sarcopenia, which comprise one or more short-chain or medium-chain fatty acids; or an alkyl ester thereof, as an active ingredient.

Another aspect of the present invention provides a composition for the diagnosis of a possible sarcopenia group or sarcopenia group, which comprises, as an active ingredient, an agent capable of measuring the content of one or more short-chain or medium-chain fatty acids in the blood.

Still another aspect of the present invention provides a method for providing information for determining a possible sarcopenia group or sarcopenia group, which comprises (i) collecting blood from a subject and measuring the content of one or more short-chain or medium-chain fatty acids; (ii) dividing the measured value in step (i) above by the content value of one or more short-chain or medium-chain fatty acids in the plasma of a normal control group; and (iii) when the divided value above is less than 1, determining that the subject is in the state of a possible sarcopenia group or sarcopenia group.

Still another aspect of the present invention provides a method for treating sarcopenia, which comprises administering to a subject one or more short-chain or medium-chain fatty acids; or one or more esters or salts of these fatty acids.

Still another aspect of the present invention provides a use of one or more short-chain or medium-chain fatty acids; or esters or salts of these fatty acids in the preparation of a pharmaceutical composition for preventing, treating, or improving sarcopenia.

Still another aspect of the present invention provides a use of one or more short-chain or medium-chain fatty acids; or esters or salts of these fatty acids for preventing or treating sarcopenia.

### Advantageous Effects of Invention

The composition of the present invention comprising short-chain or medium-chain fatty acids has the effect of preventing, treating, or improving sarcopenia, and can be usefully used as a sarcopenia treatment or health supplement, and has the advantage that it allows for accurate and easy diagnosis of sarcopenia through a method of providing information for determining the possible sarcopenia group or sarcopenia group.

However, the effects of the present invention are not limited to the effects mentioned above, and other effects not mentioned will be clearly understood by those skilled in the art from the following description.

### Brief Description of Drawings

Fig. 1 shows the sarcopenia diagnosis algorithm according to Asian Working Group for Sarcopenia (AWGS) 2019. There are several diagnostic criteria for sarcopenia worldwide, but in Asia, sarcopenia is diagnosed in accordance with the sarcopenia diagnostic algorithm established by the AWGS.
Fig. 2 shows the contents of butyrate/isobutyrate, valerate, caproate, and heptanoate in the plasma of the possible sarcopenia group and sarcopenia group compared to the normal control group. The contents were converted to ln, and Welch's *t*-test was performed. * means p<0.05, ** means p<0.01, *** means p<0.005, and # means p<0.0001.
Fig. 3 shows receiver operating characteristic (ROC) curves and area under curve (AUC) values for butyrate/isobutyrate, valerate, caproate, and heptanoate in the possible sarcopenia group and sarcopenia group.
Fig. 4 shows changes in grip strength as a result of feeding ethyl valerate, ethyl caproate, ethyl heptanoate, ethyl octanoate, ethyl palmitate, glyceryl trivalerate, glyceryl tricaproate, and glyceryl triheptanoate to aged mice over a period of four weeks, which are the result of Mann-Whitney U test. *, # means p<0.05; **, $$ means p<0.01; ***, $$$ means p<0.001; and **** means p<0.0001.
Fig. 5 shows the changes in running time after feeding in the same manner as in Fig. 4, which are the result of the Mann-Whitney U test. * means p<0.05; and $$ means p<0.01.
Fig. 6 shows the changes in grip strength that occurred as a result of feeding diets containing inositol or inositol-diheptanoate to aged mice over a period of four weeks, which are the result of the Mann-Whitney U test. *** means p = 0.0003.

### Best Mode for Carrying out the Invention

Hereinafter, the present invention will be described in detail.

### 1. Pharmaceutical composition, method of treatment, and use for preventing or treating sarcopenia

An aspect of the present invention provides a pharmaceutical composition for preventing or treating sarcopenia, which comprises one or more short-chain or medium-chain fatty acids as an active ingredient.

The "short-chain or medium-chain fatty acid" of the present invention may refer to any one selected from the group consisting of fatty acids having a chain of 1 to 12, 1 to 10, 1 to 8, 5 to 8, or 5 to 7 carbon atoms. For example, the short-chain fatty acid may refer to one or more selected from fatty acids comprising 1, 2, 3, 4, or 5 carbon atoms, and the medium-chain fatty acid may refer to one or more selected from fatty acids comprising 6, 7, 8, 9, 10, 11, or 12 carbon atoms. Additionally, the short-chain or medium-chain fatty acid may refer to any saturated, unsaturated, or branched form of a fatty acid having a chain of the above-mentioned number of carbon atoms.

More specifically, the short-chain or medium-chain fatty acid may be one or more selected from the group consisting of fatty acids having a chain of 5, 6, and 7 carbon atoms.

According to an embodiment of the present invention, the short-chain or medium-chain fatty acid may be one or more fatty acids selected from the group consisting of valerate, caproate, and heptanoate. The pharmaceutical composition of the present invention may comprise one or more, two or more, three or more, or four or more of the short-chain or medium-chain fatty acids.

The "fatty acid ester" of the present invention can improve the inherent odor of fatty acids and thereby enhance the convenience of ingestion compared to when the composition of the present invention is ingested in the form of a fatty acid.

The "fatty acid ester" of the present invention may be any one selected from the group consisting of glycerol fatty acid ester, alkyl fatty acid ester, sugar fatty acid ester, and sugar alcohol fatty acid ester.

Additionally, the ester of the fatty acid may be selected from the group consisting of methyl fatty acid ester, ethyl fatty acid ester, propyl fatty acid ester, butyl fatty acid ester, inositol fatty acid ester, and triglyceride, but other than these, any ester may be applied without limitation as long as it can increase the convenience of intake.

Additionally, the "sugar alcohol" in the "sugar alcohol fatty acid ester" may be any one selected from the group consisting of erythritol, threitol, xylitol, arabitol, ribitol, mannitol, sorbitol, galactitol, fusitol, iditol, inositol, bolemitol, isomalt, maltitol, lactitol and polyglycitol, but is not limited thereto.

In the present invention, the triglyceride of short-chain or medium-chain fatty acids may refer to one in which three molecules of the same type of fatty acids are bound to glycerol. For example, the triglyceride form of valerate or "glyceryl trivalerate" is one in which three molecules of valerate are bound to a glycerol molecule. Additionally, the triglyceride of the short-chain or medium-chain fatty acid may refer to one in which three molecules of different types of fatty acids are bonded to glycerol. For example, fatty acids having chains of 5, 6, or 7 carbon atoms may be bound one by one to one glycerol molecule.

In the present invention, the ester form of fatty acid can be absorbed into the body after being hydrolyzed by esterase or lipase in the body into a fatty acid residue and an alcohol residue, a fatty acid residue and a sugar or sugar alcohol residue, or a fatty acid residue and a glycerol residue. In this way, fatty acid esters may be understood as a means to improve the inherent bad odor of fatty acids, thereby increasing the convenience of ingestion and transporting fatty acids smoothly into the body.

"Sarcopenia", for which the pharmaceutical composition of the present invention is expected to have a preventive or therapeutic effect, may refer to one or more of the symptoms exhibited by a population selected from the "possible sarcopenia group", in which one or more of muscle strength and physical activity ability are reduced compared to the normal group according to the AWGS 2019 sarcopenia diagnostic algorithm, and the "sarcopenia group" in which muscle mass is reduced and muscle strength or physical activity ability is deteriorated. That is, the pharmaceutical composition of the present invention can be achieved by improving one or more selected from the group consisting of muscle mass, muscle strength, and ability of physical activity in a subject in need of prevention or treatment of sarcopenia. In addition, the improvement effect of the pharmaceutical composition of the present invention is not limited to Asians, and the excellent effect of the present invention is not affected by factors such as race, physical characteristics, and sex.

In the present invention, the term "prevention" is used to encompass, without limitation, any and all actions that inhibit or delay the symptoms of reduction of muscle mass, decrease of muscle strength, and impairment of physical activity associated with sarcopenia. In the present invention, the term "treatment" is used to encompass, without limitation, any and all actions that improve or beneficially change the above-mentioned symptoms related to sarcopenia.

The short-chain or medium-chain fatty acids of the present invention; or the esters or salts of these fatty acids, may be administered at a concentration level ranging from 0.1 wt% to 100 wt% based on the total weight of the pharmaceutical composition, or even when administered at a concentration level outside the above-identified range, may be administered without limitation as long as the amount is sufficient to achieve the desired effect. For example, the short-chain or medium-chain fatty acid of the present invention may be present in an amount of 0.1 wt% to 100 wt%, 0.1 wt% to 90 wt%, 0.1 wt% to 80 wt%, 0.1 wt% to 70 wt%, 0.1 wt% to 60 wt%, 0.1 wt% to 50 wt%, 0.1 wt% to 40 wt%, 0.1 wt% to 30 wt%, 0.1 wt% to 20 wt%, 0.1 wt% to 10 wt%, 1 wt% to 100 wt%, 1 wt% to 90 wt%, 1 wt% to 80 wt%, 1 wt% to 70 wt%, 1 wt% to 60 wt%, 1 wt% to 50 wt%, 1 wt% to 40 wt%, 1 wt% to 30 wt%, 1 wt% to 20 wt%, 1 wt% to 10 wt%, 10 wt% to 100 wt%, 20 wt% to 100 wt%, 30 wt% to 100 wt%, 40 wt% to 100 wt%, 50 wt% to 100 wt%, 60 wt% to 100 wt%, 70 wt% to 100 wt%, 80 wt% to 100 wt%, or 90 wt% to 100 wt%, relative to the total weight of pharmaceutical composition.

Specifically, the amount of the fatty acid varies depending on the patient's weight, age, sex, health condition, diet, administration time, administration method, excretion rate, severity of disease, *etc.* The pharmaceutical composition of the present invention may be administered once a day or several times a day, and the administration period may be from 1 day to 12 months, but may be administered without limitation until the preventive or therapeutic effect of sarcopenia appears. For example, the short-chain or medium-chain fatty acid; or the ester or salt of these fatty acids may be administered to a male adult in an amount of 500 mg to 9000 mg, 500 mg to 8000 mg, 500 mg to 7000 mg, 500 mg to 6000 mg, 500 mg to 5000 mg, 500 mg to 4000 mg, 500 mg to 3000 mg, 500 mg to 2000 mg, 500 mg to 1000 mg, 1000 mg to 9000 mg, 1000 mg to 8000 mg, 1000 mg to 7000 mg, 1000 mg to 6000 mg, 1000 mg to 5000 mg, 1000 mg to 4000 mg, 1000 mg to 3000 mg, 1000 mg to 2000 mg, 2000 mg to 9000 mg, 2000 mg to 8000 mg, 2000 mg to 7000 mg, 2000 mg to 6000 mg, 2000 mg to 5000 mg, 2000 mg to 4000 mg, or 2000 mg to 3000 mg, but is not limited thereto, and even when administered in an amount more or less than the above-identified range, it may be administered without limitation as long as the amount is sufficient to achieve the desired effect.

The pharmaceutical composition comprising the short-chain or medium-chain fatty acid of the present invention as an active ingredient may be formulated in the form of oral dosage formulations (*e.g*., powders, granules, tablets, capsules, tablets, suspensions, emulsions, syrups, aerosols, *etc*.), preparations for external use, suppositories, or sterile injection solutions according to conventional methods and used.

The pharmaceutical composition of the present invention comprising the short-chain or medium-chain fatty acid as an active ingredient may further comprise an appropriate carrier, excipient, or diluent according to a conventional method. In detail, the pharmaceutical composition may be prepared using diluents or excipients, such as fillers, extenders, binders, wetting agents, disintegrants, and surfactants, which are commonly used in preparing formulations. Solid preparations for oral administration comprise tablets, pills, powders, granules, capsules, *etc.,* and these solid preparations may be prepared by mixing one or more excipients with the compound, such as starch, calcium carbonate, sucrose, lactose, gelatin, *etc.* Additionally, in addition to simple excipients, lubricants such as magnesium stearate and talc may also be used. Liquid preparations for oral administration may include suspensions, liquid preparations for internal use, emulsions, syrups, *etc.* In addition to the commonly used simple diluents such as water and liquid paraffin, various excipients such as wetting agents, sweeteners, fragrances, and preservatives may be comprised. Preparations for parenteral administration comprise sterile aqueous solutions, non-aqueous solutions, suspensions, emulsions, lyophilized preparations, and suppositories. As non-aqueous solvents and suspensions, propylene glycol, polyethylene glycol, vegetable oils such as olive oil, and injectable esters such as ethyl oleate may be used. As a base for suppositories, wethepsol, macrogol, Tween 61, cacao butter, laurin butter, glycerogelatin, *etc.* may be used.

The pharmaceutical composition of the present invention comprising the short-chain or medium-chain fatty acid as an active ingredient may be administered to mammals, such as rats, mice, livestock, and humans through various routes. Any mode of administration may be administered, for example by intradermal, oral, rectal, intravenous, intraperitoneal, intramuscular, subcutaneous, endometrial injection or intracerebroventricular injection, and it may preferably be administered via the oral or intravenous route, but is not limited thereto.

The administration may further comprise one or more active ingredients that exhibit the same or similar functions. For administration, one or more additional pharmaceutically acceptable carriers may be comprised. As the pharmaceutically acceptable carrier, a saline solution, sterile water, Ringer's solution, a buffered saline solution, a dextrose solution, a maltodextrin solution, glycerol, ethanol, and a mixture of one or more of these ingredients may be mixed and used, and if necessary, other common additives such as antioxidants, buffer solutions, bacteriostatic agents may be added. In addition, the compound according to the present invention may be easily formulated in various preparations, for example, by additionally adding diluents, dispersants, surfactants, binders, and lubricants to form injectable formulations (*e.g*., aqueous solutions, suspensions, emulsions, *etc*.), powders, tablets, capsules, pills, granules, or injection solutions.

According to an embodiment of the present invention, when muscle strength was measured at a time-point after 4 weeks of feeding short-chain, medium-chain, or long-chain fatty acids to aged mice, it was observed that muscle strength and motor function were improved in the mice fed with short-chain or medium-chain fatty acids, which are the type of fatty acids whose content in the plasma of sarcopenia patients was reduced, compared to the muscle strength of mice fed with fatty acids whose content in the plasma of sarcopenia patients was not reduced. When the short-chain or medium-chain fatty acid of the present invention was administered, the effect of treating or improving sarcopenia was observed *(see* Example 2). Therefore, the composition of the present invention may be used as a therapeutic agent for preventing or treating sarcopenia by enhancing muscle strength and motor function.

Another aspect of the present invention provides a method for treating sarcopenia, which comprises administering to a subject one or more short-chain or medium-chain fatty acids; or one or more esters or salts of these fatty acids.

Still another aspect of the present invention provides a use of one or more short-chain or medium-chain fatty acids; or esters or salts of these fatty acids, in preparation of a pharmaceutical composition for preventing, treating, or improving sarcopenia.

Still another aspect of the present invention provides a use of one or more short-chain or medium-chain fatty acids; or esters or salts of these fatty acids for treating or preventing sarcopenia. All terms relating to the treatment method and use in the present invention may be understood to be the same as the terms defined with respect to the composition above.

### 2. Health functional food composition and health functional food for preventing or improving sarcopenia

An aspect of the present invention provides a health functional food composition for preventing or improving sarcopenia, which comprises short-chain or medium-chain fatty acids as an active ingredient.

The health functional food composition and health functional food of the present invention compriseone or more short-chain or medium-chain fatty acids, or esters or salts of these fatty acids as an active ingredient.

In this regard, the description regarding "short-chain or medium-chain fatty acid", "ester of fatty acid", "sarcopenia", and "prevention" are as described in the section '1. Pharmaceutical composition, method of treatment, and use for preventing or treating sarcopenia' above.

The "health functional food" of the present invention refers to a food, which is prepared by adding a short-chain or medium-chain fatty acid to food materials (e.g., beverages, teas, spices, gums, confectioneries, *etc*.) or by encapsulation, powderization, into suspensions, *etc.* and in which the consumption of the same brings about a specific effect in terms of health, but unlike general drugs, it has the advantage in that it does not cause any side effects that may occur when taking drugs for a long period of time because it is made from food. Since the health functional food of the present invention, obtained in this way, can be consumed on a daily basis, it can be ingested easily and conveniently and is thus very useful, as it is expected to prevent or improve sarcopenia.

The "food composition" of the present invention may refer to a composition that can be used to produce the above-mentioned health functional food.

There is no particular limitation on the type of "food" of the present invention as long as it the short-chain or medium-chain fatty acid of the present invention can be added thereto. Examples of the food to which the fatty acid of the present invention can be added include meats, sausages, breads, chocolates, candies, snacks, confectioneries, pizzas, ramens, other noodles, gums, dairy products comprising ice cream, various soups, beverages, teas, drinks, alcoholic beverages, vitamin complexes, *etc.*

The food or health functional food of the present invention may comprise various aromatic agents or natural carbohydrates, various nutritional supplements, vitamins, electrolytes, flavoring agents, colorants, pectic acid and salts thereof, alginic acid and salts thereof, organic acids, protective colloidal thickeners, pH adjusters, stabilizers, preservatives, glycerin, alcohol, carbonating agents used in carbonated drinks, *etc.* as additional ingredients.

In the present invention, "improvement" is used to refer, without limitation, to all actions that improve parameters related to the treatment of sarcopenia, and may refer, for example, to actions that at least reduce the degree of symptoms.

Accordingly, the food or health functional food composition of the present invention comprises one or more short-chain or medium-chain fatty acids, esters or salts of these fatty acids, as an active ingredient.

The short-chain or medium-chain fatty acid may be selected from the group consisting of fatty acids having a chain of 5, 6 and 7 carbon atoms. For example, the short-chain or medium-chain fatty acid may be any one selected from the group consisting of valerate, caproate, and heptanoate.

In addition, the ester of the fatty acid may be any one selected from the group consisting of glycerol fatty acid ester, alkyl fatty acid ester, sugar fatty acid ester, and sugar alcohol fatty acid ester. In addition, the ester may be selected from the group consisting of methyl fatty acid ester, ethyl fatty acid ester, propyl fatty acid ester, butyl fatty acid ester, inositol fatty acid ester, and triglyceride.

### 3. Composition for diagnosis of possible sarcopenia group or sarcopenia group

An aspect of the present invention provides a composition for diagnosing a possible sarcopenia group or sarcopenia group, which comprises as an active ingredient an agent capable of measuring the content of short-chain or medium-chain fatty acids in the blood.

The agent comprised in the diagnostic composition of the present invention can measure the content of one or more short-chain or medium-chain fatty acids in the blood.

In the present invention, the one or more short-chain or medium-chain fatty acids in the blood whose content is to be measured may be one or more fatty acids selected from the group consisting of fatty acids having a chain of 5, 6, and 7 carbon atoms.

In this regard, the description regarding "short-chain or medium-chain fatty acid" are as described in the section '1. Pharmaceutical composition, method of treatment, and use for preventing or treating sarcopenia' above.

In the present invention, "measuring the content" of short-chain or medium-chain fatty acids in the blood means measuring the content of short-chain or medium-chain fatty acids comprised in the blood, which may mean absolute content or relative content, for example, in the present invention, mass spectrometry may be used as a method to measure the content of short-chain or medium-chain fatty acids. In addition, "agent capable of measuring content" may refer, without limitation, to an agent capable of measuring the absolute or relative content of short-chain or medium-chain fatty acids comprised in the blood. The measuring the content of short-chain or medium-chain fatty acids in the blood as in the present invention has the advantage in that it can reflect various information regarding the biochemical change process in the body that has occurred up to the moment immediately before blood collection, thereby enabling early diagnosis of sarcopenia.

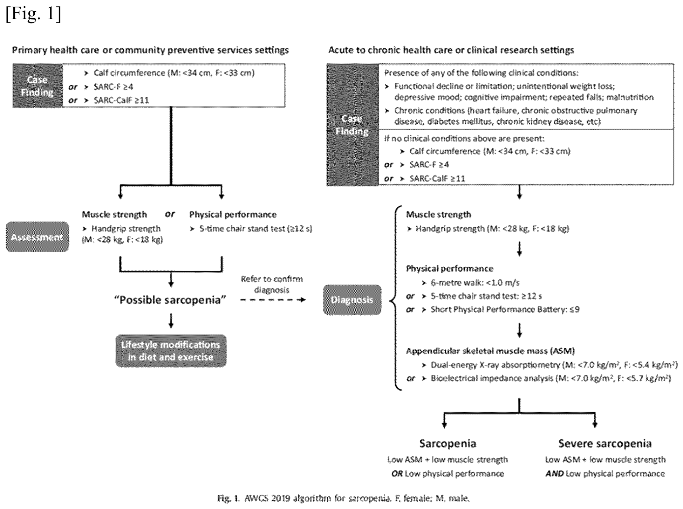

The "possible sarcopenia group" and "sarcopenia group" of the present invention may refer to groups classified according to the AWGS 2019 sarcopenia diagnosis algorithms. Specifically, the "possible sarcopenia group" refers to a group, in which one or more of muscle strength and ability of physical activity are reduced compared to the normal group, and refers to a group of patients in whom symptoms of sarcopenia have begun to progress. In addition, "sarcopenia group" refers to a group, which shows a decrease of muscle mass, and deterioration of muscle strength and ability of physical activity compared to the normal group, and refers to a group of patients who clearly show the symptoms of sarcopenia *(see* Fig. 1).

In the present invention, the term "diagnosis" refers to confirming the presence or characteristics of pathological conditions, and for the purpose of the present invention, it refers to not only the classification into a normal group, a possible sarcopenia group, and a sarcopenia group depending on the degree of sarcopenia progression, but also comprehensive determination of the degree of treatment or improvement of sarcopenia, responsiveness and resistance to drugs, *etc.* in the corresponding subject after treatment of sarcopenia.

### 4. Method of providing information for determination of possible sarcopenia group or sarcopenia group

An aspect of the present invention provides a method of providing information for determining a subject as a possible sarcopenia group or sarcopenia group by measuring the content of short-chain or medium-chain fatty acids in the subject's blood.

In the information provision method of the present invention, the description of "short-chain or medium-chain fatty acid", "measurement of content", "possible sarcopenia group", and "sarcopenia group" are as described in the sections '1. Pharmaceutical composition, method of treatment, and use for preventing or treating sarcopenia' and '3. Composition for diagnosis of possible sarcopenia group or sarcopenia group' above.

The method of providing information for determining a possible sarcopenia group, possible sarcopenia group or sarcopenia group of the present invention comprises: (i) collecting blood from a subject and measuring the content of one or more short-chain or medium-chain fatty acids in the plasma; (ii) dividing the measured value in step (i) above by the content value of one or more short-chain or medium-chain fatty acids in the plasma of a normal control group; and (iii) when the divided value is less than 1, determining that the subject is in the state of a possible sarcopenia group or sarcopenia group.

In an embodiment, it was observed that the content of short-chain or medium-chain fatty acids in the plasma of the subject is lower than that of short-chain or medium-chain fatty acids in the plasma of a normal control group. Accordingly, the method for providing information in the present invention comprisescomparing the content of short-chain or medium-chain fatty acids in the plasma of a subject and a normal control group. The case where the value divided in step (iii) above is less than 1 refers to a case in which the value is 0.90 or less, 0.85 or less, 0.80 or less, 0.75 or less, 0.70 or less, 0.65 or less, 0.60 or less, 0.55 or less, 0.50 or less, or 0.45 or less.

More specifically, according to one embodiment of the present invention, the content of valerate may be 0.50 or less, 0.47 or less, or 0.45 or less in the possible sarcopenia group compared to the normal control group, and may be 0.45 or less, 0.42 or less, or 0.40 or less in the sarcopenia group. The content of caproate may be 0.83 or less, 0.80 or less, or 0.77 or less in the possible sarcopenia group compared to the normal control group, and may be 0.87 or less, 0.85 or less, or 0.82 or less in the sarcopenia group. The content of heptanoate may be 0.55 or less, 0.53 or less, or 0.50 or less in the possible sarcopenia group compared to the normal control group, and may be 0.57 or less, 0.55 or less, or 0.54 or less in the sarcopenia group *(see* Example 1-1).

More specifically, according to one embodiment of the present invention, when patients were diagnosed as one of the normal control group, possible sarcopenia group, and sarcopenia group based on the content of short-chain or medium-chain fatty acids in the plasma, the AUC value of the ROC curve was calculated to determine whether the classification of patients was consistent with the classification in accordance with AWGS 2019 sarcopenia diagnostic algorithm. In particular, the value for butyrate or isobutyrate may be 0.77 or less, 0.75 or less, or 0.73 or less in the possible sarcopenia group, and 0.75 or less, 0.73 or less, or 0.71 or less in the sarcopenia group. The values for valerate may be 0.85 or less, 0.83 or less, or 0.81 or less in the possible sarcopenia group, and 0.87 or less, 0.85 or less, or 0.84 or less in the sarcopenia group. The values for caproate could be 0.85 or less, 0.83 or less, or 0.82 or less in the possible sarcopenia group, and 0.85 or less, 0.80 or less, or 0.78 or less in the sarcopenia group. The values for heptanoate may be 0.97 or less, 0.95 or less, or 0.94 or less in the possible sarcopenia group, and 0.95 or less, 0.93 or less, or 0.92 or less in the sarcopenia group *(see* Example 1-2).

### [Examples]

Hereinafter, the present invention will be described in more detail by the following examples. However, the following examples are provided to facilitate a better understanding of the present invention, and the content of the present invention is not limited by these examples.

### Example 1. Diagnosis of sarcopenia using short-chain or medium-chain fatty acid content

### Example 1-1. Determination of short-chain or medium-chain fatty acid content in blood of patients with sarcopenia

In order to diagnose sarcopenia by measuring the content of short-chain or medium-chain fatty acids in the blood, plasma was separated from the blood of the normal control group and the sarcopenia group, respectively, and the content of short-chain and medium-chain fatty acids was measured.

The sarcopenia groups were divided into two groups (*i.e.,* a possible sarcopenia group and a sarcopenia group) in accordance with Asian Working Group for Sarcopenia (AWGS) 2019 sarcopenia diagnosis algorithm, and an experiment was performed on 90 men aged 70 or older in each group. Descriptions of the symptoms of each group classified in accordance with the AWGS sarcopenia diagnosis algorithm are as listed in the table below.

**[Table 1]**

| Classification | Symptoms | No. of Target People |
|---|---|---|
| Normal | muscle mass, muscle strength, and physical activity ability are all normal | 30 |
| Possible sarcopenia | deterioration of one or more of muscle strength and physical activity ability | 30 |
| Sarcopenia | decrease of muscle mass, and deterioration of muscle strength or physical activity ability | 30 |

The AWGS 2019 sarcopenia diagnosis algorithm and the determination criteria for symptoms listed in Table 1 above are shown in Fig. 1, and in this experiment targeting men, the experimental groups were divided according to the reference values for limb skeletal muscle mass and hand grip strength for men as shown in Fig. 1. Specifically, muscle mass was measured as appendicular skeletal muscle mass (ASM), and when ASM (kg)/(height (m))² was < 7.0 kg/m², it was determined that muscle mass decreased. Muscle strength was measured by handgrip strength, and when handgrip strength was <28 kg, it was determined that muscle strength was decreased. Finally, physical activity ability was determined by walking speed (6-metre walk), time required to sit and stand up 5 times (5-time chair stand test), or a physical function index. It was determined that physical activity ability was reduced when walking speed was <1.0 m/s, time required to sit down and stand up 5 times was ≥ 12s, or physical function index (Short Physical Performance Battery; SPPB) was ≤ 9. The SPPB is a test standard developed by the US National Institutes of Health that can determine sarcopenia by comprehensively testing walking speed, sitting and standing, grip strength, *etc.*

First, mass spectrometry was performed on a total of four kinds of fatty acids, *i.e.,* butyrate/isobutyrate and valerate (short-chain fatty acids) and caproate and heptanoate (medium-chain fatty acids) in the plasma of the normal control group, possible sarcopenia group, and sarcopenia group.

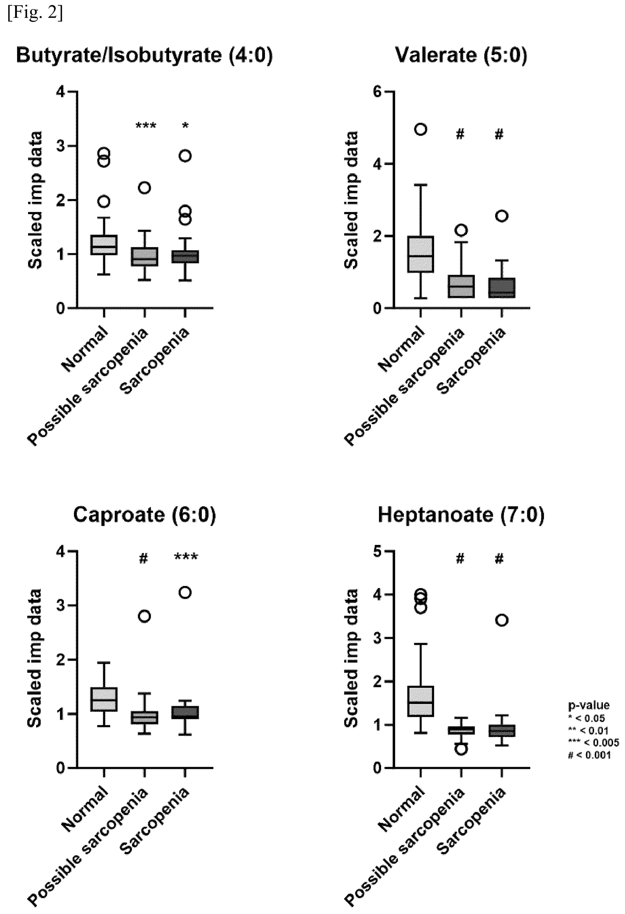

The content of each fatty acid in the plasma of the possible sarcopenia group, and sarcopenia group was measured through mass spectrometry. The median value of the measured raw data was set to 1, and based on the same, the scaled imp data, which is a value corrected for the remaining content, was calculated. Then, these corrected content values were divided by the levels of each fatty acid found in the plasma of the normal control group, and this divided values enabled the calculation of the relative levels of fatty acids in the plasma of both the possible sarcopenia group and sarcopenia group, compared to the normal control group *(see* Table 2 and Fig. 2).

**[Table 2]**

| Classification of Fatty Acids | Names of Fatty Acids | Pos-Sar | Sar |
|---|---|---|---|
| | | Normal | Normal |
| Short-Chain Fatty Acid | butyrate/isobutyrate (4:0) | 0.77 | 0.82 |
| | valerate (5:0) | 0.45 | 0.40 |
| Medium-Chain Fatty Acid | caproate (6:0) | 0.77 | 0.82 |
| | heptanoate (7:0) | 0.50 | 0.54 |

As a result, as shown in Table 2 above, it was observed that the fatty acid contents in the plasma of the possible sarcopenia group and sarcopenia group were decreased to 0.85 or less overall, compared to the normal control group.

In particular, in the case of heptanoate, a medium chain fatty acid, it was confirmed that the level was reduced to about half compared to the normal control group in the plasma of the possible sarcopenia group and sarcopenia group. Meanwhile, in the case of valerate, a short-chain fatty acid, it was confirmed that the level was reduced to less than half compared to the normal control group in the plasma of the possible sarcopenia group and the sarcopenia group. Finally, in the case of caproate, it was observed that the level was reduced to about 80% of that of the normal control group in the plasma of both the possible sarcopenia group and sarcopenia group.

### Example 1-2. Confirmation of accuracy of short-chain or medium-chain fatty acid as diagnostic marker for sarcopenia

In order to determine the accuracy of short-chain or medium-chain fatty acids as a diagnostic marker for sarcopenia, the Receiver Operating Characteristic (ROC) curve was plotted and the Area Under Curve (AUC) value was calculated.

ROC curve analysis is a method used to determine the diagnostic accuracy of a specific indicator, in which the x-axis as "1-specificity" represents a false positive rate where specificity is a value defined as true positive/(false positive + true positive), and the y-axis as "sensitivity" represents a true positive rate.

That is, in the experiment of the present invention, when the patient was diagnosed as belonging to one of the normal control group, possible sarcopenia group, and sarcopenia group according to the content of short-chain or medium-chain fatty acids in the patient's plasma, when it matched the patient's classification according to the AWGS 2019 sarcopenia diagnosis algorithm, it was determined as a true positive, whereas when it did not match, it was determined as a false positive.

The area under the curve of the ROC curve is called AUC. The closer the AUC value is to 1, the better the diagnostic accuracy of a specific indicator, and generally, a value of 0.7 or higher is considered useful as a diagnostic indicator (Table 3; Source: Muller et al., (2005). Can routine laboratory tests discriminate between severe acute respiratory syndrome and other causes of community-acquired pneumonia? Clinical Infectious Diseases 40(8):1079-1086).

**[Table 3]**

| AUC | Determination of Accuracy |
|---|---|
| 0.9 ≤ AUC < 1.0 | Excellent |
| 0.8 ≤ AUC < 0.9 | Good |
| 0.7 ≤ AUC < 0.8 | Fair |
| AUC < 0.7 | Poor |

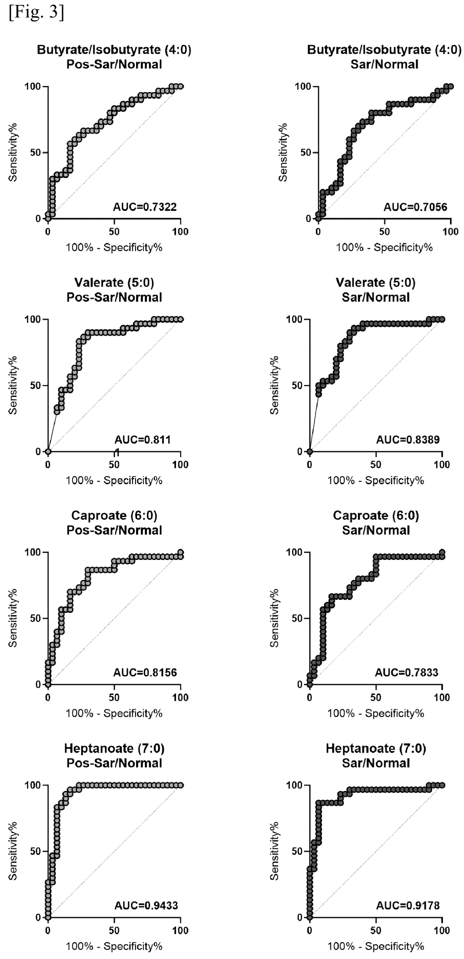

The results of calculating the AUC values of short-chain and medium-chain fatty acids according to the method described above are shown in Table 4 below and Fig. 3.

**[Table 4]**

| Classification of Fatty Acids | Names of Fatty Acids | Pos-Sar | Sar |
|---|---|---|---|
| | | Normal | Normal |
| Short-Chain Fatty Acid | butyrate/isobutyrate (4:0) | 0.73 | 0.71 |
| | valerate (5:0) | 0.81 | 0.84 |
| Medium-Chain Fatty Acid | caproate (6:0) | 0.82 | 0.78 |
| | heptanoate (7:0) | 0.94 | 0.92 |

As mentioned above, all fatty acid markers showed effective AUC values of 0.7 or more in the possible sarcopenia group and sarcopenia group, thus confirming that the diagnostic accuracy was high. Among them, particularly, heptanoate showed a high AUC value of 0.9 or more for the possible sarcopenia group and the sarcopenia group, thus confirming that the diagnostic accuracy was the best as a diagnostic marker for both the possible sarcopenia group and sarcopenia group.

### Example 2. Confirmation of sarcopenia treatment effect through intake of short-chain or medium-chain fatty acid

In order to confirm the effectiveness of treating sarcopenia through the intake of short-chain or medium-chain fatty acids, five to ten 18-20-month-old male mice were fed *ad libitum* with short-chain or medium-chain fatty acids, corresponding to 5 wt% of the feed.

### Example 2-1. Intake of ester forms of short-chain or medium-chain fatty acids

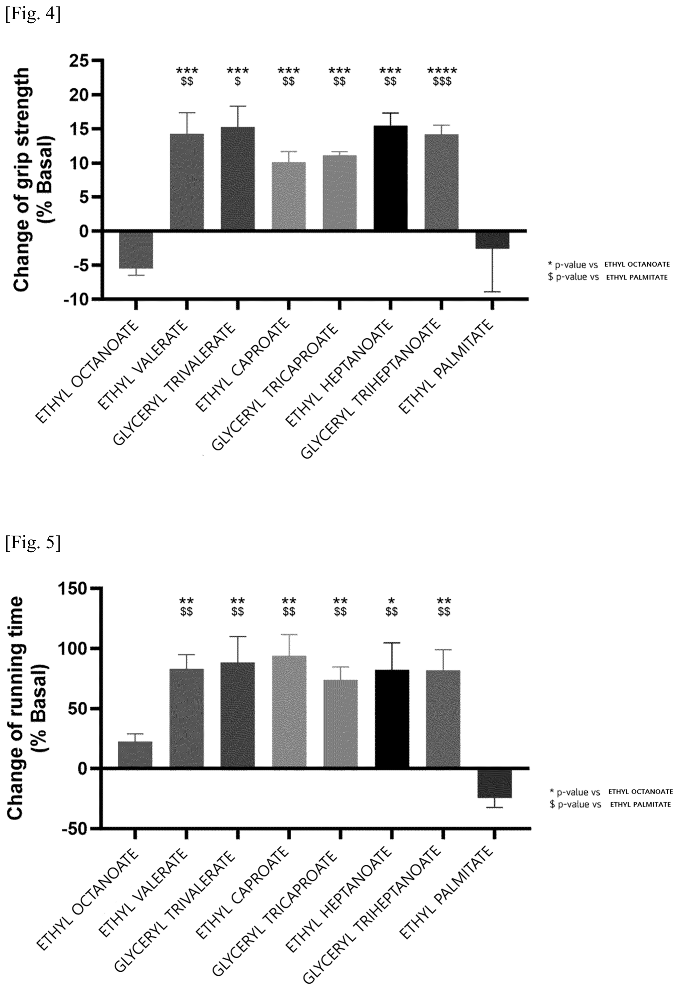

Specifically, since short-chain and medium-chain fatty acids themselves are difficult to ingest due to their bad odors, fatty acids used as flavoring agents were fed. Fatty acids were supplied in the form of ethyl esters or triglycerides through feed and were degraded by the esterase or lipase enzyme in the mouse body. After feeding as described above, the grip strength of the mice was measured using a digital force gauge (source: SHIMPO digital force gauge) 4 weeks after the start of feeding (see Table 5 and Fig. 4). In addition, a running test was performed to confirm the changes in motor function, and the change value (%) in the running time that the mice could run without stopping was compared (see Table 6 and Fig. 5).

As described in Table 5 below, "ethyl valerate", "ethyl caproate", "ethyl heptanoate", "ethyl octanoate", and "ethyl palmitate" refer to ethyl ester forms of fatty acids; and "glyceryl trivalerate", "glyceryl tricaproate", and "glyceryl triheptanoate" are tri(acyl)glyceride forms of fatty acids, in which three of the same type of fatty acids are bound to a glycerol molecule.

**[Table 5]**

| Classification of Fatty Acids | Names of Fatty Acids | Change in Grip Strength |
|---|---|---|
| | | (% Basal) Mean ± SEM |
| Short-Chain Fatty Acid | ethyl valerate (5:0) | 14.29 ± 3.092 |
| | glyceryl trivalerate (5:0) | 15.29 ± 3.061 |
| Medium-Chain Fatty Acid | ethyl caproate (6:0) | 10.13 ± 1.563 |
| | glyceryl tricaproate (6:0) | 11.17 ± 0.4773 |
| | ethyl heptanoate (7:0) | 15.50 ± 1.822 |
| | glyceryl triheptanoate (7:0) | 14.22 ± 1.299 |
| | ethyl octanoate (8:0) | -5.5 ± 0.982 |
| Long-Chain Fatty Acid | ethyl palmitate (16:0) | -2.6 ± 2.821 |

As described in Table 5 above, it was confirmed that the muscle strength of the mice fed with ethyl octanoate and ethyl palmitate, which are ester forms of octanoic acid and palmitic acid (*i.e.,* long-chain fatty acid), whose contents were not reduced in the plasma of sarcopenia patients, decreased by 5.5% and 2.6%, respectively, compared to the muscle strength before the experiment, whereas the muscle strength of the mice fed with valerate (*i.e*., short-chain fatty acid), ethyl valerate, glyceryl trivalerate, ethyl caproate, glyceryl tricaproate, ethyl heptanoate, and glyceryl triheptanoate, which are ester forms of caproic acid and heptanoic acid (*i.e.,* medium-chain fatty acids), whose contents were reduced in the plasma of sarcopenia patients, was improved by about 10.1% to 15.5%.

**[Table 6]**

| Classification of Fatty Acids | Names of Fatty Acids | Amount of Change in Running Time (% Basal) Mean ± SEM |
|---|---|---|
| Short-Chain Fatty Acid | ethyl valerate (5:0) | 83 ± 11.86 |
| | glyceryl trivalerate (5:0) | 88.25 ± 21.86 |
| Medium-Chain Fatty Acid | ethyl caproate (6:0) | 94.14 ± 17.55 |
| | glyceryl tricaproate (6:0) | 74 ± 10.67 |
| | ethyl heptanoate (7:0) | 82.25 ± 22.58 |
| | glyceryl triheptanoate (7:0) | 82.14 ± 16.96 |
| | ethyl octanoate (8:0) | 22.5 ± 6.265 |
| Long Chain Fatty Acid | ethyl palmitate (16:0) | -24.4 ± 3.586 |

Additionally, as described in Table 6 above, it was confirmed that with respect to the change in running time in the mice fed with ethyl octanoate and ethyl palmitate, which are ester forms of octanoic acid and palmitic acid (*i.e.*, long-chain fatty acid), whose contents were not reduced in the plasma of sarcopenia patients, a small change with an increase of 22.5% or a decrease of 24.4%, whereas the change in running time in the mice fed with valerate (*i.e*., short-chain fatty acid), ethyl valerate, glyceryl trivalerate, ethyl caproate, glyceryl tricaproate, ethyl heptanoate, and glyceryl triheptanoate, which are ester forms of caproic acid and heptanoic acid (*i.e.*, medium-chain fatty acids), whose contents were reduced in the plasma of sarcopenia patients, was significantly improved to 74.0% to 94.1%, thus indicating the improvement in motor function.

### Example 2-2. Intake of sugar alcohols in the form of short-chain or medium-chain fatty acid

An attempt was made to investigate the therapeutic effect of sarcopenia through the intake of fatty acids in the form of sugar alcohols. Seven to eight 18-month-old male mice were fed *ad libitum* with diets including inositol or inositol-diheptanoate fatty acids corresponding to 5 wt% of the feed, and the mice fed with only inositol served as a control group.

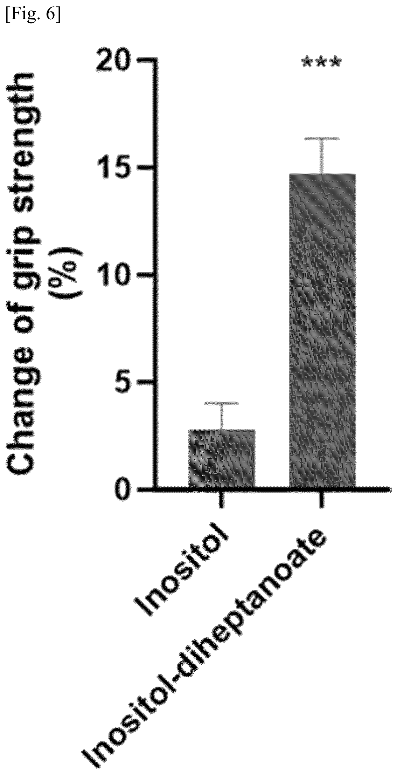

The grip strength of the mice was performed in the same manner as in Example 2-1 above *(see* Table 7 and Fig. 6).

**[Table 7]**

| Names of Fatty Acids | Change in Grip Strength |
|---|---|
| | Mean ± SEM (% Basal) |
| Inositol | 2.8 ± 1.228 |
| Inositol-diheptanoate | 14.70 ± 1.655 |

As described in Table 7 above, it was confirmed that compared to when only inositol was fed, when inositol-diheptanoate fatty acid was fed, the amount of change in grip strength over 4 weeks was improved by about 12%. Through this, it was confirmed that muscle strength was improved and increased even in the mice fed with fatty acids in the form of sugar alcohols. In this way, the composition of the present invention can be used as a therapeutic agent for preventing or treating sarcopenia by improving and strengthening muscle strength in sarcopenic patients.

## Claims

1. A pharmaceutical composition for preventing or treating sarcopenia, comprising one or more short-chain or medium-chain fatty acids; or esters or salts of these fatty acids as an active ingredient.

2. The pharmaceutical composition of claim 1, wherein the short-chain or medium-chain fatty acid is any one selected from the group consisting of fatty acids having a chain of 5, 6, or 7 carbon atoms.

3. The pharmaceutical composition of claim 1, wherein the ester of the fatty acid is any one selected from the group consisting of glycerol fatty acid ester, alkyl fatty acid ester, sugar fatty acid ester, and sugar alcohol fatty acid ester.

4. The pharmaceutical composition of claim 1, wherein the short-chain or medium-chain fatty acid is any one selected from the group consisting of valerate, caproate, and heptanoate.

5. The pharmaceutical composition of claim 1, wherein the ester of the short-chain or medium-chain fatty acid is selected from the group consisting of methyl fatty acid ester, ethyl fatty acid ester, propyl fatty acid ester, butyl fatty acid ester, inositol fatty acid ester, and triglyceride.

6. A health functional food composition for preventing or improving sarcopenia, comprising one or more short-chain or medium-chain fatty acids; or esters or salts of these fatty acids as an active ingredient.

7. The health functional food composition of claim 6, wherein the short-chain or medium-chain fatty acid is any one selected from the group consisting of fatty acids having a chain of 5, 6, or 7 carbon atoms.

8. The health functional food composition of claim 6, wherein the ester of the fatty acid is any one selected from the group consisting of glycerol fatty acid ester, alkyl fatty acid ester, sugar fatty acid ester, and sugar alcohol fatty acid ester.

9. The health functional food composition of claim 6, wherein the short-chain or medium-chain fatty acid is any one selected from the group consisting of valerate, caproate, and heptanoate.

10. The health functional food composition of claim 6, wherein the ester of the short-chain or medium-chain fatty acid is selected from the group consisting of methyl fatty acid ester, ethyl fatty acid ester, propyl fatty acid ester, butyl fatty acid ester, inositol fatty acid ester, and triglyceride.

11. A health functional food for preventing or improving sarcopenia, comprising one or more short-chain or medium-chain fatty acids; or esters or salts of these fatty acids.

12. The health functional food of claim 11, wherein the short-chain or medium-chain fatty acid is any one selected from the group consisting of fatty acids having a chain of 5, 6, or 7 carbon atoms.

13. The health functional food of claim 11, wherein the ester of the fatty acid is any one selected from the group consisting of glycerol fatty acid ester, alkyl fatty acid ester, sugar fatty acid ester, and sugar alcohol fatty acid ester.

14. The health functional food of claim 11, wherein the short-chain or medium-chain fatty acid is any one selected from the group consisting of valerate, caproate, and heptanoate.

15. The health functional food of claim 11, wherein the ester of the short-chain or medium-chain fatty acid is selected from the group consisting of methyl fatty acid ester, ethyl fatty acid ester, propyl fatty acid ester, butyl fatty acid ester, inositol fatty acid ester, and triglyceride.

16. A diagnostic composition for a possible sarcopenia group or sarcopenia group, comprising, as an active ingredient, an agent capable of measuring the content of one or more short-chain or medium-chain fatty acids in the blood.

17. The diagnostic composition of claim 16, wherein the short-chain or medium-chain fatty acids are one or more fatty acids selected from the group consisting of fatty acids having a chain of 5, 6, or 7 carbon atoms.

18. A method for providing information for determining a possible sarcopenia group or sarcopenia group, comprising:
(i) collecting blood from a subject and measuring the content of one or more short-chain or medium-chain fatty acids in a plasma;
(ii) dividing the measured value in step (i) above by the content value of one or more short-chain or medium-chain fatty acids in the plasma of a normal control group; and
(iii) when the divided value above is less than 1, determining that the subject is in the state of a possible sarcopenia group or sarcopenia group.

19. The method of claim 18, wherein the short-chain or medium-chain fatty acids are one or more fatty acids selected from the group consisting of fatty acids having a chain of 5, 6, or 7 carbon atoms.

20. The method of claim 18, wherein the divided value in step (iii) above is 0.85 or less, the subject is determined to be in the state of a possible sarcopenia group or sarcopenia group.

21. A method for treating sarcopenia, comprising administering to a subject one or more short-chain or medium-chain fatty acids; or one or more esters or salts of these fatty acids.

22. Use of one or more short-chain or medium-chain fatty acids; or esters or salts of these fatty acids for preparing a pharmaceutical composition for preventing, treating, or improving sarcopenia.

23. Use of one or more short-chain or medium-chain fatty acids; or esters or salts of these fatty acids for preventing or treating sarcopenia.
